(19) Europäisches Patentamt
European Patent Office
Office européen des brevets

(11) EP 1 013 277 A1

(12) **EUROPEAN PATENT APPLICATION**
published in accordance with Art. 158(3) EPC

(43) Date of publication:
**28.06.2000 Bulletin 2000/26**

(21) Application number: **98931096.6**

(22) Date of filing: **14.07.1998**

(51) Int. Cl.[7]: **A61K 31/555**, A61K 9/14

(86) International application number:
**PCT/JP98/03154**

(87) International publication number:
**WO 00/03718 (27.01.2000 Gazette 2000/04)**

(84) Designated Contracting States:
**DE FR GB IT**

(71) Applicant:
**TORAY INDUSTRIES, INC.**
**Tokyo 103-8666 (JP)**

(72) Inventors:
- **AKIMOTO, Masahiro**
  **Nagoya-shi, Aichi 455-0021 (JP)**
- **TAMURA, Fuminori**
  **Mishima-shi, Shizuoka 411-0033 (JP)**
- **OZAWA, Yumi**
  **Numazu-shi, Shizuoka 410-0002 (JP)**

(74) Representative: **Coleiro, Raymond**
**MEWBURN ELLIS**
**York House**
**23 Kingsway**
**London WC2B 6HP (GB)**

(54) **FREEZE-DRIED PREPARATIONS AND PROCESS FOR PRODUCING THE SAME**

(57) A freeze-dried product containing a platinum (II) complex represented by the following general formula as a primary component.

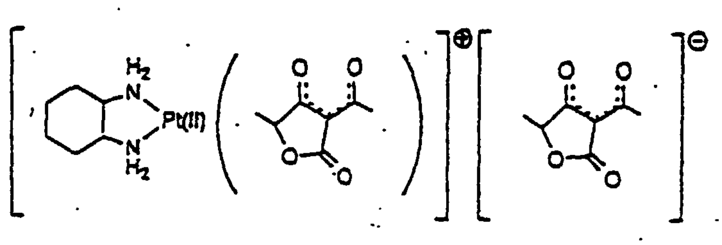

The freeze-dried product according to the present invention has superior storage stability, and handling thereof for administration in medical treatments is facilitated due to rapid dissolution thereof in water for injection. In addition, they are characterized in requiring no special storage conditions such as refrigeration and freezing.

EP 1 013 277 A1

## Description

Technical Field

The present invention relates to a freeze-dried product composed of a platinum (II) complex as a primary component.

Background Art

As platinum agents for treatment of malignant tumors, cisplatin, carboplatin, and the like are well known; however, these compounds have side effects, such as renal toxicity, bone marrow toxicity, or the like. Those compounds are supplied for medical treatments as aqueous solutions, saline solution, saccharide solutions, and/or freeze-dried powders, and they are then administered by intravenous injection after dilution thereof with diluents. However, since these compounds have low solubility and poor dissolution when diluted, problems occur in that large quantities of diluents are necessary, splash-back occur during dissolution at the time of use, and the like, are known (Japanese Unexamined Patent Application Publication No. 7-53368).

A platinum (II) complex, represented by the following general formula (I)

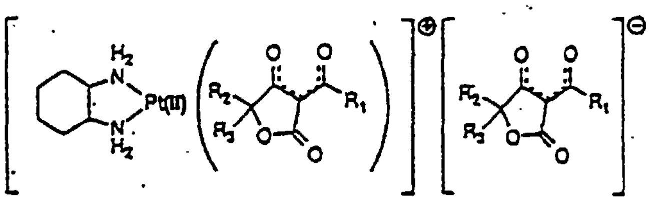

(I)

wherein $R^1$ represents a lower hydrocarbon radical having 1 to 3 carbon atoms; $R^2$ and $R^3$ each represents a hydrogen atom or a lower hydrocarbon radical having 1 to 3 carbon atoms; and the configuration of 1,2-diaminocyclohexane is cis-, trans-l-, or trans-d-,
which is the primary component of the present invention, is effective for treating malignant tumors, in a manner similar to that of cisplatin, carboplatin, and the like, and is anticipated to reduce side effects (Chem. Lett., pages 529 to 532, 1993), an appearance of the platinum (II) complex being a lemon-yellow or white crystalline powder. The platinum (II) complex was conventionally available supplied in the form of aqueous solutions or powder filling products.

However, when the aqueous solutions described above were administered by injection, even though handling thereof was facilitated in medical treatments, physical and chemical stability in long-term storage were not satisfactory. In addition, the powder filling products are superior in terms of long-term storage stability; however, dissolution thereof was not sufficiently rapid during dissolution at the time for administration, and handling must be attentively performed since shaking or stirring operations are required.

An object of the present invention is to solve the problems described above.

Disclosure of Invention

The present invention provides freeze-dried products composed of a platinum (II) complex as primary component and a method for preparing the same, the platinum (II) complex being represented by the following general formula (I),

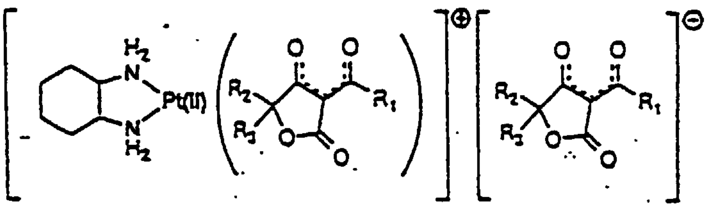

(I)

wherein $R^1$ represents a lower hydrocarbon radical having 1 to 3 carbon atoms; $R^2$ and $R^3$ each represents a hydrogen atom or a lower hydrocarbon radical having 1 to 3 carbon atoms; and the configuration of 1,2-diaminocyclohexane is cis-, trans-l-, or trans-d-.

Best Mode for Carrying Out the Invention

The present invention provides freeze-dried products composed of a platinum (II) complex represented by the general formula (I) described above as a primary component and a method for preparing the same.

In $R^1$, $R^2$, and $R^3$ in the formula (I) described above, lower hydrocarbon radicals having 1 to 3 carbon atoms are preferably alkyl groups and alkenyl groups, and examples thereof are methyl, ethyl, propyl, vinyl, isopropyl, allyl, and isopropenyl groups.

A particularly preferable platinum (II) complex represented by the general formula (I) is [(5S)-3-acetyl-5-methyl-2,4(3H,5H)-furandionato-$O^3$,$O^4$][(1R,2R)-cyclohexanediamine-N,N'] platinum(1+)(5S)-3-acetyl-5-methyl-2,4(3H,5H)-furandione enolate (hereinafter referred to as "compound 1") as shown in the following formula.

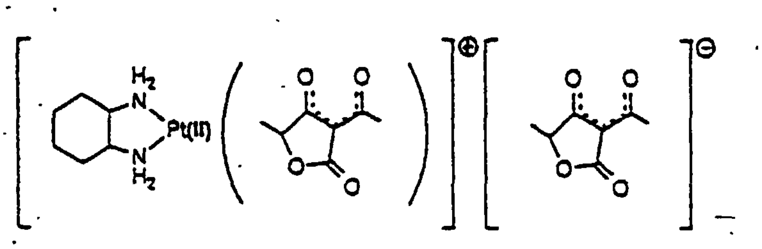

In the present invention, isomers or derivatives of the general formula (I) may be used as primary components. The derivatives mentioned above include, solvates, complexes, hydrates, geometrical isomers, and products having substituted anion portion as shown in a general formula (II) described below, or the like, in which $R^1$ represents a lower hydrocarbon radical having 1 to 3 carbon atoms; $R^2$ and $R^3$ each represents a hydrogen atom or a lower hydrocarbon radical having 1 to 3 carbon atoms; the configuration of 1,2-diaminocyclohexane is cis-, trans-l-, or trans-d-; and $X^-$ represents an anion of an inorganic acid or an organic carboxylic acid.

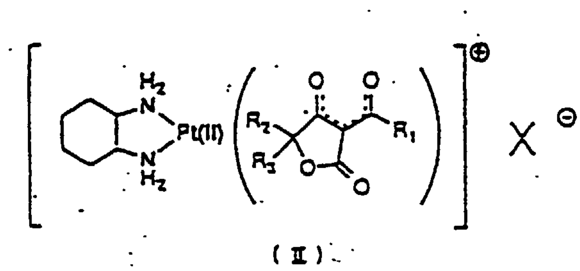

(II)

The platinum (II) complexes as the primary components used in the present invention may be prepared by the methods disclosed in USP 5,302,587 and International Patent Publication No. WO 95/28,408.

Usefulness of the platinum (II) complexes as the primary components used in the present invention for agents for treatment of malignant tumors was reported in USP 5,302,587, International Patent Publication No. WO 95/28,408, and Chem. Lett., pages 529 to 532, 1993, and the freeze-dried products according to the present invention can be used as agents for treatment of malignant tumors: however, this is not so limited.

In the freeze-dried products according to the present invention, the content of the platinum (II) complex as a primary component is preferably 50 to 500 mg per dosage unit, and more preferably, 100 to 300 mg. The freeze-dried products according to the present invention may only be composed of the primary components as described above; however, if necessary, pharmaceutically acceptable excipients may be compounded therewith. The usefulness according to the present invention is not affected by compounding excipients. Excipients may be, for example, lactose, mannitol, glucose, maltose, dextran, and cellulose derivatives. The excipient content is preferably 50 to 200 percent by weight of the primary component, and more preferably, 50 to 100 percent by weight.

The products according to the present invention may be prepared, for example, by the following method. First, the platinum (II) complex as a primary component is dissolved in a solvent. Solvents to be used are preferably

sterile water (water for injection), a lower alcohol, or a mixture thereof, and more preferably, is sterile water (water for injection). The content of the primary component is preferably 0.5 to 5 (weight/volume)%, and more preferably, is 1 to 3%. In this case, excipients may be added when necessary.

Next, the solution is sterilized by filtration using a membrane filter having a pore diameter of 0.2 µm, and a part of the solution equivalent to 50 to 500 mg of the solid weight of the primary component is poured into a container, such as a vial. The vial made of a hard glass or a plastic for medical use and having a volume of 5 to 100 ml, may be used.

The vial containing a part of the solution containing the primary component is fitted with a rubber stopper and is placed in a vacuum freeze-drying apparatus. A shelf temperature is rapidly cooled to -50 to -30°C, and the vial is frozen for 5 to 15 hours. Preferably, the shelf temperature is maintained at -40°C for not less than 10 hours. Next, the shelf temperature is set to be 25°C, and the solvent is then sublimated at a pressure of 5 to 15 pascals. After the sublimation is nearly complete, a resulting product is then dehydrated at a pressure of not more than 1.5 pascals for 5 to 20 hours. In view of product yield, the time for dehydration at a pressure of not more than 1.5 pascals is preferably not less than 8 hours. Taking cost, workability, and the like into consideration, dehydration exceeding 20 hours nay be optionally determined. After the process at a pressure of not more than 1.5 pascals for a predetermined period of time, dehydration for a long period of time at a shelf temperature of not less thin 25°C may be performed, so long as the platinum (II) complex as the primary component is not adversely affected.

After the completion of the dehydration, the vial is completely sealed with a rubber stopper and is covered with an aluminum cap, whereby the freeze-dried product is obtained.

As described above, one of the preparing methods according to the present invention comprises the steps of rapidly freezing to -50 to -30°C, and dehydrating by two stages, at a pressure of 5 to 15 pascals and at a pressure of not more than 1.5 pascals.

Next, features of the freeze-dried product according to the present invention will be described.

Water content of the freeze-dried product according to the present invention is specified by the following method. Generally, 2-methoxy ethanol is added to the vial containing 50 to 500 mg of the platinum (II) complex as the primary component so that the concentration thereof is 1 to 10%. The entire solution is recovered, for example, by an injection syringe, and an amount of water ($W_H$) µg is obtained by a water content analysis using the Karl Fisher method (coulometric titration). When necessary, a sum of the amounts of water ($W_H$) can be obtained by repeating the same treatments two to five times for one vial. In order to perform each quantity measurement, blank measurement of 2-methoxy ethanol ($W_B$) is performed to adjust the amount of water ($W_H$), and the water content is calculated in accordance with the following equation. An apparatus for coulometric Karl-Fisher titration AQ-7 (HIRANUMA SANGYOU Co., Ltd.) or the like is used as a Karl Fisher type water content titration apparatus. A solvent for analyzing ketone derivatives is used as a test solvent.

$$\text{Water content (\%)} = \sum_{i=1}^{5} (W_H - W_B)i / (\text{interior weight content per vial} \times 10)$$

The stability of the platinum (II) complex in a product is influenced by water contained therein; in conditions in which the temperature is not less than 40°C, higher than room temperature, stability of the platinum (II) complex is decreased and degradation thereof is accelerated. In the present invention, in order to maintain the stability of the platinum (II) complex, a water content is preferably not more than 2.5 percent by weight of the platinum (II) complex in one vial of the freeze-dried product. In addition, taking seasonal variation in room temperature into consideration, a water content of not more than 1 percent by weight is more preferable to obtain long-term stability.

Dissolution of the freeze-dried product according to the present invention is specified by the following method. Generally, 10 ml of water for injection is gently added using an injection syringe, a whole pipette, or the like, in the vial containing 50 to 500 mg of the platinum (II) complex as the primary component. In the condition in which the vial is allowed to stand undisturbed, the time immediately from the addition of the water for injection to the complete dissolution of the contents in the vial is measured. In the present invention, the time required for the dissolution is preferably not more than 120 seconds, more preferably, not more than 60 seconds, and even more preferably, not more than 30 seconds.

When freeze-dried products are administered by injection, it is necessary for freeze-dried products to dissolve in a solvent, such as in water for injection, before use, and it is preferable for freeze-dried products to dissolve quickly after adding the solvent. Quick dissolution of freeze-dried products or ready-dissolution thereof guarantees potency and safety. Since the freeze-dried products according to the present invention are quickly dissolved, there is no risk of administrating intravenously platinum (II) complex containing non-dissolved crystal thereof.

The stability in the present invention is the stability in terms of chemical and physical characteristics.

Because of progressive decomposition of products caused by thermal stresses, remaining amounts of primary components decrease and significant discoloration of the appearance of the products can be observed, whereby remaining amounts are indicators of stability in terms of chemical characteristics. Slight discoloration observed when stresses are applied to the product at elevated temperatures will not indicate serious reduction in titer of the primary component and the potency thereof. However, use of significantly discolored products is not desirable since clouding may occur when products are dissolved, and precipitation thereof may occur. In terms of physical stability, progressive changes in form is an indicator; when the stability is inferior, changes in the form, such as shrinkage of the contents in products, can be observed at not less than room temperature.

The remaining rate is specified by the following method. Generally, a vial containing 50 to 500 mg of the primary component is sealed and stored in an environment with a temperature between room temperature and 60°C. After storing the vial at a predetermined temperature for a predetermined time, the total contents in the vial are dissolved by adding water so that the concentration of the primary component is between 0.5 to 2%. The concentration of the primary component is obtained by high-performance liquid chromatography (HPLC) measurement, and the remaining rate (%) versus the concentration at the beginning of storage is calculated by the following equation.

$$\text{Remaining rate (\%)} = (\text{concentration of primary component at the end of storage/concentration of primary component at the beginning of storage}) \times 100$$

In order to provide this product for medical treatments, it is preferable that when the product is to be stored at room temperature for one year, the remaining rate be not less than 95%, and no shrinkage of the form of the content and no significant discoloration occur, more preferably, when the product is to be stored at room temperature for at least 2 years, the remaining rate be not less than 95%, and no shrinkage of the form of the content and no significant discoloration occur. In addition, it is preferable that when the product is to be stored at 40°C for 6 months, the remaining rate be not less than 95%, and no shrinkage of the form of the content and no significant discoloration occur. Specifically, in order to maintain long-term stability, it it preferable that when the product is to be stored at 60°C for 2 months, the remaining rate be not less thin 95%, and no shrinkage of the form of the content and no significant discoloration occur. The freeze-dried product according to the present invention has a higher remaining rate than that of aqueous solutions and the storage stability is therefore significantly improved.

Since the freeze-dried product according to the present invention has superior storage stability, storage thereof at room temperature is possible and special storage, such as refrigeration or freezing, is not required. In addition, since the product is readily dissolved in solvents, handling thereof for administration in medical treatments is facilitated, and in particular, the product may be effectively used in an injection. The solvent used for the product for administration by injection is preferably water for injection. A product diluted in a mixture composed of materials that are pharmaceutically acceptable in terms of having no adverse effects on the potency and the stability of platinum (II) complex as the primary component, may be administered. The mixture may be composed of, for example, water and a polyalkylene glycol, and/or a saccharide solution. Polyalkylene glycols to be used may be a polyethylene glycol and the like, and saccharides to be used may be glucose, mannitol, and the like.

## EXAMPLES

Hereinbelow, the present invention will be specifically described with reference to Examples; however, the invention is not limited thereto.

### EXAMPLE 1

Preparation of Freeze-dried Product for the Evaluation of Long-Term Stability:

In accordance with the following procedure, product 1, product 2 according to the present invention, and comparative product 1 were obtained.

Product 1

"Compound 1" was dissolved in an amount of 10 mg per 1 ml of water for injection. The entire solution of "compound 1" was sterilized by filtration using a membrane filter having a pore diameter of 0.2 μm (Millipak® 20, Nihon Millipore Ltd.)

Each filtrate in an amount of 10 ml was separately poured into a vial (V-TR20CS, Fuji GLASS Co., Ltd.) having a volume of 20 ml, each vial was fitted with a rubber stopper (V10-F8W, KK Daikyo Seiko), and each vial was then

placed in a vacuum freeze-drying apparatus (Edwards-Kniese, 2.3 $m^2$ shelf surface (com8032), Edwards-Kniese).

The shelf temperature of the freeze-drying apparatus was rapidly reduced from room temperature to approximately - 40°C and was maintained for 10 hours. Next, the shelf temperature was controlled to be 25°C, and water was sublimated at a pressure of 13 pascals. After temperatures of the filtrates became equal to that of the shelf, the filtrates were dehydrated at a pressure of 1.3 pascals for 20 hours, and then the vials were completely sealed. The vials were removed and were covered with aluminum caps (20FLIP CAP, ISHIDA PRESS KOGYO K.K.), and the freeze-dried product was thus obtained.

"Compound 1" in an amount of 100 mg was contained in the product in one vial, and the water content of the product was 0.8 percent by weight.

Product 2

"Compound 1" was dissolved in an amount of 10 mg per 1 ml of water for injection. The entire solution of "compound 1" was sterilized by filtration using a membrane filter having a pore diameter of 0.2 µm (Millipak® 20, Nihon Millipore Ltd.)

Each filtrate in an amount of 10 ml was separately poured into a vial (V-TR20CS, Fuji GLASS Co., Ltd.) having a volume of 20 ml, each vial was fitted with a rubber stopper (V10-F8W, KK Daikyo Seiko), and each vial was then placed in a vacuum freeze-drying apparatus (VIRTIS SUBLIMATOR MODEL25-SRC-MS, VerTis Company Inc.).

The shelf temperature of the freeze-drying apparatus was rapidly reduced from room temperature to approximately - 40°C and was maintained for 10 hours. Next, the shelf temperature was controlled to be 25°C, and water was sublimated at a pressure of 13 pascals. After the temperatures of the filtrates became equal to that of the shelf, the filtrates were dehydrated at a pressure of 1.3 pascals for 20 hours, and then the vials were completely sealed. The vials were removed and were covered with aluminum caps (20FLIP CAP, ISHIDA PRESS KOGYO K.K.), and the freeze-dried product was thus obtained.

"Compound 1" in an amount of 100 mg was contained in the product in one vial, and the water content of the product was 2.5 percent by weight. Comparative Product 1

"Compound 1" was dissolved in an amount of 10 mg per 1 ml of water for injection. The entire solution of "compound 1" was sterilized by filtration using, a membrane filter having a pore diameter of 0.2 µm (Fluorodyne® IIDFP, Nihon Pall Ltd.)

Each filtrate in an amount of 10 ml was separately poured into a vial (V-TR20CS, Fuji GLASS Co., Ltd.) having a volume of 20 ml, each vial was completely sealed with a rubber stopper (V10-F8W, KK Daikyo Seiko), and each vial was then covered with an aluminum cap (20FLIP CAP, ISHIDA PRESS KOGYO K.K.).

The obtained product was a product containing "compound 1" in an amount of 10 ml, the concentration thereof being 10 mg/ml.

EXAMPLE 2

Long-term Stability of Freeze-Dried Product:

Product 1, product 2, and comparative product 1 were stored at room temperature or at 40°C, and changes in appearances of the contents of the products and the remaining rates of "compound 1" were evaluated for long-term stability. Concentration measurements by HPLC were performed under the following conditions. The results are shown in Table 1.

| | |
|---|---|
| Column: | CAPCELL PAK C18 SG-120 (4.6×150mm) |
| Measurement: | 230 nm |
| Injection volume: | 10 µl |
| Mobile phase: | Distilled water 90%/methanol 10%/acetic acid being added corresponding to 0.1% |
| Carrier rate: | 1 ml/min |
| Temperature: | 40°C |
| Internal standard: | 2,6 dimethyl-γ-pyrrone aqueous solution (0.15 weight/volume %) |
| Sample concentration: | approximately 0.6 mg/ml |

Table 1

| Storage stability of freeze-dried product | | | | |
|---|---|---|---|---|
| | Water content (%) | Storage conditions | Appearance after storage | Remaining rate (%) |
| Product 1 | 0.8 | 25°C, 2 years | No change | 100 |
| Product 2 | 2.5 | 40°C, 6 months | Slightly yellowed | 98 |
| Comparative product 1 | - | 40°C, 6 months | Yellowed | 94 |

As can be seen from Table 1, regarding product 1, no changes in appearance after storage for 2 years at 25°C was observed and the remaining rate was 100%. Regarding product 2, slight yellow discoloration was observed after storage for 6 months at 40°C; however, the remaining rate was 98%. The storage stability of both products was superior. In contrast, regarding comparative product 1, the colorless and clear solution turned to yellow after storage for 6 months at 40°C and the remaining rate was 94%, so that the stability thereof was significantly inferior.

EXAMPLE 3

Preparation of Freeze-Dried Product for Evaluation to Confirm the Influence of Water Content:

In accordance with the following procedure, products 3 to 5, and comparative products 2 and 3 were obtained.

Product 3

"Compound 1" was dissolved in an amount of 10 mg per 1 ml of water for injection. The entire solution of "compound 1" was sterilized by filtration using a membrane filter having a pore diameter of 0.2 µm (Fluorodyne® IIDFP, Nihon Pall Ltd.)

Each filtrate in an amount of 10 ml was separately poured into a vial (V-TR20CS, Fuji GLASS Co., Ltd.) having a volume of 20 ml, each vial was fitted with a rubber stopper (V10-F8W, KK Daikyo Seiko), and each vial was then placed in a vacuum freeze-drying apparatus (DFB-2030-1MS-ST/CIP, ULVAC Japan Ltd.).

The shelf temperature of the freeze-drying apparatus was rapidly reduced from room temperature to approximately - 40°C and was maintained for 10 hours. Next, the shelf temperature was controlled to be 25°C and water was sublimated at a pressure of 13 pascals. After temperatures of the filtrates became equal to that of the shelf, the filtrates were dehydrated at a pressure of 1.3 pascals for 8 hours, and the vials were then completely sealed. The vials were removed and were covered with aluminum caps (20FLIP CAP, ISHIDA PRESS KOGYO K.K.), and the freeze-dried product was thus obtained.

"Compound 1" in an amount of 100 mg was contained in the product in one vial, and the water content of the product was 0.3 percent by weight.

Product 4

"Compound 1" was dissolved in an amount of 30 mg per 1 ml of water for injection, and product 4 was prepared by following processes similar to those described for Product 3. However, dehydration thereof was performed at a pressure of 1.3 pascals for 8 hours after temperature of the product became equal to that of the shelf.

"Compound 1" in an amount of 300 mg was contained in the product in one vial, and the water content of the product was 0.2 percent by weight.

Product 5

"Compound 1" in an amount of 10 mg and mannitol in an amount of 10 mg were dissolved per 1 ml of water for injection, and product 5 was prepated by following processes similar to those described for Product 3. However, dehydration thereof was performed at a pressure of 1.3 pascals for 18 hours after temperature of the product became equal to that of the shelf.

"Compound 1" in an amount of 100 mg was contained in the product in one vial, and the water content of

the product was 0.2 percent by weight.

Comparative product 2

"Compound 1" in an amount of 10 mg and lactose in an amount of 10 mg were dissolved per 1 ml of water for injection, and comparative product 2 was prepared by following processes similar to those described for Product 3. However, dehydration thereof was only performed at a pressure of 13 pascals, and 2-stage dehydration was not performed.

"Compound 1" in an amount of 100 mg was contained in the product in one vial, and the water content of the product was 2.4 percent by weight. Comparative product 3

"Compound 1" was dissolved in an amount of 30 mg per 1 ml of water for injection, and comparative product 3 was prepared by following processes similar to those described for Product 3. However, dehydration thereof was performed at a pressure of 1.3 pascals for 4 hours after the temperature of the product became equal to that of the shelf.

"Compound 1" in an amount of 300 mg was contained in the product in one vial, and a water content of the product was 3.8 percent by weight.

Example 4

Effect of Water Content on the Stability of Freeze-Dried Product:

After storing the freeze-dried products according to the present invention at 60°C for 2 months, the effects of water content on stability were evaluated. Evaluation of storage stability of the products described below at 60°C was performed; appearances of the products and remaining rates thereof were evaluated. Concentration measurement by HPLC was performed in accordance with the following method. The results are shown in Table 2.

| | |
|---|---|
| Column: | YMC PAK CN A-503 (4.6×150mm) |
| Measurement: | 230 nm |
| Injection volume: | 5 µl |
| Mobile phase: | pH 7 phosphate buffer solution 90% /acetonitrile 10% |
| Carrier rate: | 1 ml/min |
| Temperature: | 40°C |
| Internal standard: | 2,6 dimethyl-γ-pyrrone aqueous solution (0.2 weight/volume %) |
| Sample concentration: | approximately 0.4 mg/ml |

Table 2

| Effects of Water Content on Stability (storage at 60°C for 2 months) | | | | | |
|---|---|---|---|---|---|
| | Content (mg) | Method for Product | Water Content (%) | Form, Color Change | Remaining rate (%) |
| Product 2 | 100 | 2-stage | 2.5 | No change, Slightly yellowed | 98 |
| Product 3 | 100 | 2-stage | 0.3 | No change, Slightly yellowed | 100 |
| Product 4 | 300 | 2-stage | 0.2 | No change, Slightly yellowed | 101 |
| Product 5 | 100 | 2-stage | 0.2 | No change, Slightly yellowed | 99 |
| Comparative Product 2 | 100 | 1-stage | 2.4 | Shrinkage, yellowed | 94 |
| Comparative Product 3 | 300 | 2-stage | 3.8 | Shrinkage, Ochered | 98 |
| Notes in the column of Method for Product are as follows.<br>2-stage: 2-stage dehydration at pressures of 13 pascals and at pressures of 1.3 pascals<br>1-stage: Dehydration only at a pressure of 13 pascals | | | | | |

As can be seen in Table 2, products 2 to 5, which were processed by 2-stage dehydration and had water contents of not more than 2.5 percent by weight, showed the remaining rates of 98 to 101% under conditions of 60°C after 2 months, and changes in the form of the products were not observed. In contrast, the remaining rate of comparative product 2 processed by 1-stage dehydration was decreased to 94%, even the water content thereof was 2.4 percent by weight, and shrinkage of the form was observed. The remaining rate of comparative product 3 having the water content of 3.8 percent by weight was 98 percent; however, the color thereof became ocher and the form shrank to an extreme degree, and as a result, the stability was somewhat inferior.

In contrast, it was confirmed that the remaining rate in storage conditions of 60°C after 20 days of comparative product 1, which was the water-soluble product, was decreased to 90%, and the solution thereof was discolored to yellow. Therefore, significant improvement of stability was seen by freeze-drying the product.

Example 5

Effects according to the Present Invention on Solubility

Solubility of five pieces of each of the freeze-dried products, that is, product 1, 3, 4, and 5, were evaluated. Comparative product 3 and comparative product 4 obtained by the method described below were used as comparisons. The results are shown in Table 3.

Comparative product 4

"Compound 1" in an amount of 100 mg was stored in a vial having a volume of 20 ml as a comparative product without treatments.

Table 3

| Solubility of Freeze-dried Product | | | | | | |
|---|---|---|---|---|---|---|
| | Content (mg) | Dissolution Time (seconds) | | | | |
| | | 1 | 2 | 3 | 4 | 5 |
| Product 1 | 100 | 9 | 9 | 9 | 11 | 15 |
| Product 3 | 100 | 11 | 12 | 8 | 16 | 9 |
| Product 4 | 300 | 15 | 12 | 18 | 23 | 21 |
| Product 5 | 100 | 17 | 15 | 21 | 18 | 25 |
| Comparative Product 3 | 100 | 105 | 25 | 47 | 28 | 33 |
| Comparative Product 4 | 300 | 90 | 47 | >300 | 154 | >300 |

As can be seen from Table 3, products 1, 3, 4, and 5, all exhibited superior dissolution properties. Comparative product 3 having a relatively high water content of 3.8 percent by weight, had variation of solubility; that is, some were superior and others were somewhat inferior; however, significantly superior dissolution was observed compared to comparative product 4 filled with "compound 1" without treatments. Regarding comparative product 4, there was large variation in dissolution, and some exceeding 300 seconds were observed.

Industrial Applicability

Freeze-dried product according to the present invention has superior storage stability, and handling thereof for administration in medical treatments is facilitated due to rapid dissolution thereof in water for injection.

**Claims**

1. A freeze-dried product comprising a platinum (II) complex, represented by the following general formula (I) as primary component,

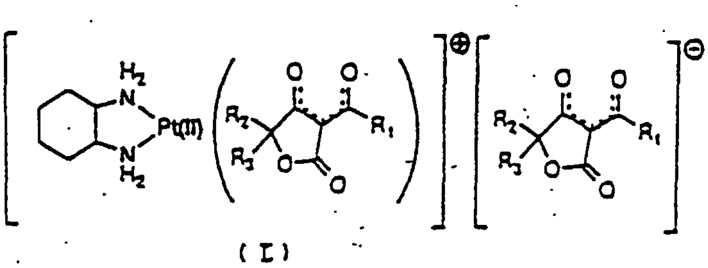

( I )

wherein $R^1$ represents a lower hydrocarbon radical having 1 to 3 carbon atoms; $R^2$ and $R^3$ each represents a hydrogen atom or a lower hydrocarbon radical having 1 to 3 carbon atoms; and the configuration of 1,2-diaminocyclohexane is cis-, trans-l-, or trans-d-.

2. A freeze-dried product according to Claim 1, prepared by a process comprising the steps of:

rapidly freezing a solution containing the platinum (II) complex to -50 to -30°C, and
dehydrating the solution in two stages, at a pressure of 5 to 15 pascals and at a pressure of not more than 1.5 pascals.

3. A freeze-dried product according to Claim 1 or Claim 2, wherein the water content is not more than 2.5 percent by weight.

4. A freeze-dried product according to Claims 1 to 3, wherein the platinum (II) complex in an amount of 50 to 500 mg is contained in one dosage unit of the freeze-dried product.

5. A freeze-dried product according to Claims 1 to 4, wherein the platinum (II) complex stored at room temperature for at least two years has a remaining rate of not less than 95 percent, and no change in shrinkage of the form and no significant discoloration of the freeze-dried product occur.

6. A method for preparing a freeze-dried product comprising the steps of:

rapidly freezing a solution containing a platinum (II) complex represented by the following general formula (I) to -50 to -30°C, and
dehydrating the solution in two steps, at a pressure of 5 to 15 pascals and at a pressure of not more than 1.5 pascals:

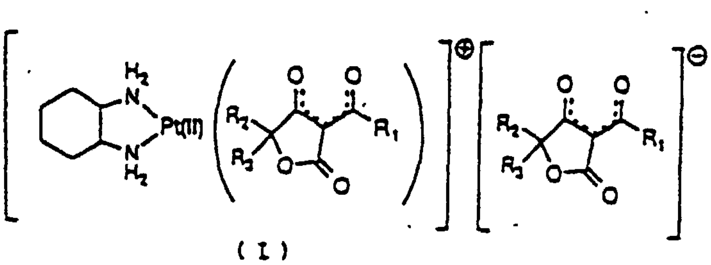

( I )

wherein $R^1$ represents a lower hydrocarbon radical having 1 to 3 carbon atoms; $R^2$ and $R^3$ each represents a hydrogen atom or a lower hydrocarbon radical having 1 to 3 carbon atoms; and the configuration of 1,2-diaminocyclohexane is cis-, trans-l-, or trans-d-.

7. A method for preparing a freeze-dried product according to Claim 6, wherein the concentration of the platinum (II) complex in the solution is between 0.5 to 5 (weight/volume) percent.

## INTERNATIONAL SEARCH REPORT

International application No.
PCT/JP98/03154

A. CLASSIFICATION OF SUBJECT MATTER
Int.Cl[6] A61K31/555, A61K9/14

According to International Patent Classification (IPC) or to both national classification and IPC

B. FIELDS SEARCHED

Minimum documentation searched (classification system followed by classification symbols)
Int.Cl[6] A61K31/555, A61K9/14

Documentation searched other than minimum documentation to the extent that such documents are included in the fields searched

Electronic data base consulted during the international search (name of data base and, where practicable, search terms used)
CA (STN)

C. DOCUMENTS CONSIDERED TO BE RELEVANT

| Category* | Citation of document, with indication, where appropriate, of the relevant passages | Relevant to claim No. |
|---|---|---|
| PX | JP, 10-194974, A (Toray Industries, Inc.), 28 July, 1998 (28. 07. 98) | 1-7 |
| Y | WO, 91/09041, A1 (Toray Industries, Inc.), 27 June, 1991 (27. 06. 91), Claims & EP, 457921, A1 & US, 5302587, A | 1-7 |
| Y | "Recent Technology of Manufacturing Pharmaceutical Preparations and its Application. I", Osaka: K.K. Iyaku Janarusha, 1 September, 1983 (01. 09. 83), p.74-77 | 1-7 |
| Y | JP, 7-112994, A (Asta Medica AG.), 2 May, 1995 (02. 05. 95), Claims ; page 13, left column, lines 21 to 30 ; page 28, left column, lines 31 to 44, right column, lines 19 to 31 & EP, 451753, A1 & US, 5194644, A & US, 5238955, A | 1-7 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 September, 1998 (14. 09. 98) | 29 September, 1998 (29. 09. 98) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP98/03154

C (Continuation). DOCUMENTS CONSIDERED TO BE RELEVANT

| Category* | Citation of document, with indication, where appropriate, of the relevant passages | Relevant to claim No. |
|---|---|---|
| Y | JP, 4-169531, A (Sumitomo Pharmaceuticals Co., Ltd.), 17 June, 1992 (17. 06. 92), Claims ; page 3, lower right column, lines 8 to 12 (Family: none) | 1-7 |
| Y | JP, 2-157291, A (Morishita Pharmaceutical Co., Ltd.), 18 June, 1990 (18. 06. 90), Claims ; page 3, lower left column, lines 5 to 7 (Family: none) | 1-7 |

Form PCT/ISA/210 (continuation of second sheet) (July 1992)